# FASCICULE DE BREVET EUROPEEN

(11) **EP 4 380 431 B1**
(45) Date de publication et mention de la délivrance du brevet: **09.09.2026**
(21) Numéro de dépôt: 22754125.7
(22) Date de dépôt: 20.07.2022
(51) Int. Cl.: A61B 5/00

(54) **ANALYSE QUANTITATIVE DE FLUCTUATIONS DANS DES TISSUS BIOLOGIQUES PAR IMAGERIE PHOTOACOUSTIQUE MULTISPECTRALE**
QUANTITATIVE ANALYSE VON SCHWANKUNGEN IN BIOLOGISCHEN GEWEBEN DURCH MULTISPEKTRALE FOTOAKUSTISCHE BILDGEBUNG
QUANTATIVE ANALYSIS OF FLUCTUATIONS IN BIOLOGICAL TISSUES VIA MULTISPECTRAL PHOTOACOUSTIC IMAGING

(30) Priorité: 03.08.2021 FR 2108452
(43) Date de publication de la demande: 12.06.2024
(73) Titulaire: UNIVERSITÉ GRENOBLE ALPES, 38400 Saint-Martin-d'Heres (FR); Centre National de la Recherche Scientifique, 75016 Paris (FR)
(72) Inventeur: ARNAL, Bastien, 38000 GRENOBLE (FR); BOSSY, Emmanuel, 38700 CORENC (FR); GODEFROY, Guillaume, 91800 BRUNOY (FR)
(74) Mandataire: Cabinet Chaillot
(86) Numéro de dépôt international: PCT/FR2022/051444
(87) Numéro de publication internationale: WO 2023/012416

(56) Documents cités:
- SERGEY VILOV ET AL: "Photoacoustic fluctuation imaging: theory and application to blood flow imaging", ARXIV.ORG, CORNELL UNIVERSITY LIBRARY, 201 OLIN LIBRARY CORNELL UNIVERSITY ITHACA, NY 14853, 18 November 2020 (2020-11-18), XP081816499, DOI: 10.1364/OPTICA.400517
- SERGEY VILOV ET AL: "A unified framework for photoacoustic fluctuation imaging. Application to visibility enhancement with fluctuations induced by blood flow", ARXIV.ORG, CORNELL UNIVERSITY LIBRARY, 201 OLIN LIBRARY CORNELL UNIVERSITY ITHACA, NY 14853, 16 June 2020 (2020-06-16), XP081707980
- HILL EMMA R. ET AL: "Identification and removal of laser-induced noise in photoacoustic imaging using singular value decomposition", vol. 8, no. 1, 1 January 2017 (2017-01-01), United States, pages 68, XP055916737, ISSN: 2156-7085, Retrieved from the Internet <URL:https://www.ncbi.nlm.nih.gov/pmc/articles/PMC5231316/pdf/68.pdf> DOI: 10.1364/BOE.8.000068
- AL MUKADDIM RASHID ET AL: "Enhancement of in vivo cardiac photoacoustic signal specificity using spatiotemporal singular value decomposition", JOURNAL OF BIOMEDICAL OPTICS, SPIE, 1000 20TH ST. BELLINGHAM WA 98225-6705 USA, vol. 26, no. 4, 1 April 2021 (2021-04-01), pages 46001, XP060139942, ISSN: 1083-3668, [retrieved on 20210419], DOI: 10.1117/1.JBO.26.4.046001

## Description

### Domaine technique

Le présent document concerne l'imagerie photoacoustique à résolution acoustique et plus précisément un procédé de traitement d'images acquises par un système d'imagerie photoacoustique multispectrale et un dispositif associé.

### Arrière-plan technique

La technique d'imagerie photoacoustique (appelée également opto-acoustique) est basée sur la génération d'ondes ultrasonores produites dans un échantillon (typiquement un tissu biologique) par excitation en irradiant l'échantillon avec une radiation électromagnétique, typiquement des impulsions laser dans le spectre de 300 à 2000 nm, notamment dans la fenêtre de 650 à 950 nm. De façon générale, une image photoacoustique est reconstruite à partir de la mesure de signaux acoustiques engendrés par absorption de la lumière envoyée sur l'échantillon. Pour des raisons liées à l'efficacité de la génération photoacoustique, on utilise généralement pour l'imagerie biomédicale des impulsions brèves nanosecondes pour illuminer le tissu biologique à imager : l'absorption de la lumière impulsionnelle crée une élévation rapide de température dans le tissu mou qui engendre par effet thermoélastique une élévation de pression, pression qui relaxe alors en entrainant la propagation d'ondes acoustiques impulsionnelles dans le tissu biologique.

L'imagerie photoacoustique à résolution acoustique se distingue de l'imagerie photoacoustique à résolution optique en ce qu'on utilise un réseau de capteurs d'ondes ultrasonores : connaissant la vitesse du son, on peut alors reconstruire une image indiquant les amplitudes et les positions des sources de son à partir des signaux acoustiques reçus. Alors que dans l'imagerie photoacoustique à résolution optique chaque impulsion lumineuse est focalisée point par point sur la surface de l'échantillon, et on mesure le son émis en provenance de la zone focale optique par un capteur d'onde ultrasonore unique.

L'imagerie photoacoustique à résolution acoustique est une technique d'imagerie biomédicale qui fournit un contraste d'absorption optique en profondeur dans les tissus biologiques. L'imagerie photoacoustique du sang permet par exemple d'imager les vaisseaux sanguins. Le sang, via l'hémoglobine, est en effet un élément absorbeur très abondant dans le corps humain.

Les dispositifs fixes d'imagerie photoacoustique utilisant des réseaux de capteurs piézo-électriques pour la capture des ondes ultrasonores présentent cependant des limites de visibilité et certaines structures des tissus n'apparaissent pas sur les images acquises du fait de limitations des capteurs en termes de bande passante et d'ouverture numérique, lorsque le réseau de capteurs n'englobe pas le milieu imagé. L'utilisation de systèmes d'acquisition à ouverture numérique limitée et à bande passante limitée provoquent ainsi des artefacts de visibilité limitée : les vaisseaux dont l'axe est quasi-aligné à celui de la sonde sont invisibles ainsi que les vaisseaux de grande taille. En particulier, on n'a pas accès aux ondes reçues sur le côté à 90° et autour et on détecte seulement les structures vasculaires à peu près horizontales mais les structures verticales n'apparaissent pas. Pour résoudre ce problème, il est possible d'utiliser une approche tomographique et d'employer des détecteurs large-bande. Ces détecteurs non résonants ne permettent cependant pas de réaliser une imagerie ultrasonore et les approches tomographiques associées ne sont pas compatibles avec tous les problèmes cliniques et pré-cliniques.

Des approches basées sur l'intelligence artificielle avec apprentissage profond ont aussi été proposées récemment pour pallier aux problèmes de visibilité mais cela pose des problèmes de disponibilité de données d'entraînement ainsi que de généralisation.

SERGEY VILOV ET AL: "Photoacoustic fluctuation imaging: theory and application to blood flow imaging", ARXIV.ORG, CORNELL UNIVERSITY LIBRARY, 201 OLIN LIBRARY CORNELL UNIVERSITY ITHACA, NY 14853, 18 novembre 2020 (2020-11-18) décrit une méthode d'imagerie de fluctuations photoacoustiques dans laquelle une série temporelle d'images est acquise et traitée par analyse de fluctuations et par décomposition en valeurs singulières, afin d'améliorer la visibilité et d'obtenir une image liée à l'énergie absorbée.

SERGEY VILOV ET AL: "A unified framework for photoacoustic fluctuation imaging. Application to visibility enhancement with fluctuations induced by blood flow", ARXIV.ORG, CORNELL UNIVERSITY LIBRARY, 201 OLIN LIBRARY CORNELL UNIVERSITY ITHACA, NY 14853, 16 juin 2020 (2020-06-16) décrit un cadre d'imagerie de fluctuations photoacoustiques incluant l'utilisation de la décomposition en valeurs singulières pour séparer les contributions du signal et du bruit dans des séries d'images. Le bruit de détection associé aux composantes de faible valeur singulière est réduit par filtrage.

Il apparaît donc un besoin pour une solution d'imagerie de tissus biologiques mieux adaptée.

### Résumé

Selon un premier aspect, un procédé de traitement d'images photoacoustiques comprend : une obtention d'une succession temporelle d'images d'un échantillon acquises par un système d'imagerie photoacoustique pour M_{λ} longueurs d'ondes d'impulsions d'excitations avec N images acquises par longueur d'onde; un filtrage spatio-temporel multispectral par décomposition en valeurs singulières appliqué à l'ensemble des N*M_{λ} images acquises de sorte à obtenir N*M_{λ} images filtrées ; pour chaque longueur d'onde, un calcul d'une image de variance filtrée à partir des N images filtrées, un pixel de coordonnée r dans l'image de variance filtrée étant égal à la variance de la distribution des valeurs de pixels de même coordonnée r dans les images filtrées obtenues pour cette longueur d'onde ; pour chaque longueur d'onde, une correction de l'image de variance filtrée par soustraction d'une variance du bruit électronique résiduel après filtrage spatio-temporel multispectral produit par des capteurs d'ondes ultrasonores du système d'imagerie photoacoustique. L'image de variance corrigée ainsi obtenue est proportionnelle en chaque point d'image au carré de l'énergie absorbée en un point de l'espace correspondant au point d'image concerné.

Dans un ou plusieurs modes de réalisation, le procédé comprend pour chaque longueur d'onde, une détermination d'une image de fluctuations corrigée dont chaque pixel est égal à la racine carrée du pixel correspondant de l'image de variance corrigée obtenue par ladite soustraction pour la longueur d'onde considérée. L'image de fluctuations corrigée ainsi obtenue est proportionnelle en chaque point d'image à l'énergie absorbée en un point de l'espace correspondant au point d'image concerné.

Dans un ou plusieurs modes de réalisation, le procédé comprend en outre une estimation de la variance du bruit électronique résiduel produit par les capteurs d'ondes ultrasonores sur les images, la variance du bruit électronique résiduel produit par les capteurs d'ondes ultrasonores sur les images étant estimé en fonction d'une variance du bruit électronique produit dans les signaux photoacoustiques acquis en l'absence d'échantillon corrigée par une quantité de bruit éliminée par le filtrage spatio-temporel multispectral par décomposition en valeurs singulières, la quantité de bruit éliminée étant estimée sur la base des valeurs singulières correspondant aux composantes de plus basse énergie supprimées par le filtrage spatio-temporel multispectral par décomposition en valeurs singulières.

Dans un ou plusieurs modes de réalisation, le procédé comprend, pour chaque longueur d'onde considérée, une normalisation de l'image de fluctuations corrigée par une fonction de la fluence d'impulsions laser du système d'imagerie photoacoustique de sorte à obtenir une image de fluctuations d'absorption représentative des fluctuations d'absorption dues à l'échantillon.

Dans un ou plusieurs modes de réalisation, le filtrage spatio-temporel multispectral par décomposition en valeurs singulières comprend une sélection des composantes correspondantes aux valeurs singulières de plus haute énergie à supprimer et une suppression de composantes sélectionnées, la sélection étant effectuée en choisissant parmi un ensemble de valeurs d'index un index identifiant la première composante à conserver pour lequel un rapport contraste à bruit est maximal, le rapport contraste à bruit déterminé pour un index étant calculé à partir des images de variance filtrées calculées par filtrage spatio-temporel multispectral par décomposition en valeurs singulières appliquant cet index.

Dans un ou plusieurs modes de réalisation, le rapport contraste à bruit est déterminé en éliminant le contraste dû à la valeur moyenne des images acquises pour au moins une longueur d'onde.

Dans un ou plusieurs modes de réalisation, le procédé comprend un calcul d'une image du taux de saturation en oxygène à partir d'au moins deux images de fluctuations d'absorption obtenues pour au moins deux longueurs d'onde correspondantes. Le calcul peut être effectué sur la base d'un modèle exprimant pour chaque pixel de coordonnée r une relation entre une concentration totale en hémoglobine, un taux de saturation en oxygène et la valeur au pixel r de l'image de fluctuations d'absorption.

Selon un autre aspect, un dispositif de traitement d'images photoacoustiques comprend, au moins une mémoire de données comprenant des instructions de code de programme, au moins un processeur de données, le processeur de données étant configurées pour, lorsque les instructions de code de programme sont exécutées par le processeur de données, causer l'exécution par le dispositif de traitement d'images photoacoustiques d'un procédé de traitement d'images photoacoustiques selon l'un quelconque des modes de réalisation.

Selon un autre aspect, un support de données lisible par ordinateur comprend des instructions de programme informatique qui, lorsqu'elles sont exécutées par un processeur, causent l'exécution d'un procédé de traitement d'images photoacoustiques selon l'un quelconque des modes de réalisation.

Selon un autre aspect, un programme informatique comprenant des instructions de programme informatique qui, lorsqu'elles sont exécutées par un processeur, causent l'exécution d'un procédé de traitement d'images photoacoustiques selon l'un quelconque des modes de réalisation.

### Brève description des figures

D'autres caractéristiques et avantages résulteront de la description détaillée qui va suivre, effectuée sur la base de modes de réalisation et d'exemples donnés à titre illustratif et non limitatif, en faisant référence aux figures annexées dans lesquelles :
[fig. 1] représente un organigramme simplifié d'un procédé de traitement d'images acquises par un système d'imagerie photoacoustique multispectrale;
[fig. 2] illustre des aspects d'un procédé de traitement d'images acquises par un système d'imagerie photoacoustique multispectrale;
[fig. 3A] illustre des aspects d'une méthode de filtrage spatio-temporel multispectral par décomposition en valeurs singulières utilisable dans un procédé de traitement d'images acquises par un système d'imagerie photoacoustique multispectrale ;
[fig. 3B] illustre des aspects d'une méthode de filtrage spatio-temporel multispectral par décomposition en valeurs singulières utilisable dans un procédé de traitement d'images acquises par un système d'imagerie photoacoustique multispectrale ;
[fig. 4A] représente un organigramme simplifié d'une méthode de sélection de valeurs singulières utilisable lors d'un filtrage spatio-temporel multispectral par décomposition en valeurs singulières ;
[fig. 4B] illustre des aspects d'une méthode de sélection de valeurs singulières utilisable lors d'un filtrage spatio-temporel multispectral par décomposition en valeurs singulières ;
[fig. 5A] montre deux exemples d'images d'oxygénation obtenues par un procédé de traitement d'images acquises selon la présente description et acquises selon une méthode conventionnelle.
[fig. 5B] montre deux exemples d'images d'oxygénation obtenues par un procédé de traitement d'images acquises selon la présente description et acquises selon une méthode conventionnelle.

### Description détaillée

Un procédé de traitement d'images acquises par un système d'imagerie photoacoustique multispectrale utilisant un réseau de capteurs d'ondes acoustiques pour l'acquisition d'ondes ultrasonores produites dans un échantillon à imager (généralement, un tissu biologique) par excitation laser va être décrit plus en détail.

Le procédé est applicable à l'imagerie photoacoustique de tout type de fluctuations se produisant dans des tissus biologiques. L'application du procédé à l'analyse quantitative du taux d'oxygénation sanguin sera décrite à titre d'exemple non limitatif.

Dans le cadre de ce document, on parle d'image de fluctuations pour désigner une image représentative en chaque point d'image de fluctuations temporelles, chaque point d'image (ou pixel) représentant un écart type des fluctuations considérées. Une image de fluctuations peut ainsi être obtenue à partir d'une succession temporelle d'images, chaque pixel de coordonnée r dans l'image de fluctuations étant calculé à partir de l'écart type de la distribution statistique des pixels de même coordonnée r dans la succession temporelle d'images. A chaque image de fluctuations correspond une image de variance, désignant l'image de fluctuations au carré : chaque point d'image de l'image de variance représentant une variance des fluctuations considérées, c'est-à-dire une variance de la distribution statistique des pixels de même coordonnée dans la succession temporelle d'images considérée. Selon le contexte, on parlera de l'image de variance ou de l'image de fluctuations.

Dans le cadre de ce document, les images peuvent être des images 2D ou 3D. Par souci de simplification on parlera ici de manière générale de pixel d'image pour désigner un élément d'image (« picture element ») ou de point d'image. Un pixel ou point d'image correspond à un voxel dans le cas d'une image 3D. Le procédé de traitement d'images utilise une analyse quantitative multispectrale des fluctuations détectées pour un voxel d'échantillon donné à partir des différentes images acquises. Des images (généralement des images 3D) de fluctuations représentant à chaque pixel les fluctuations des valeurs des pixels représentant un voxel donné du tissu biologique sont ainsi générées pour chaque longueur d'onde. Par exemple, à chaque pixel décrivant un vaisseau sanguin, l'écoulement sanguin provoque une fluctuation d'amplitude d'une image à l'autre. En particulier, dans le cas de vaisseaux sanguins, les globules rouges n'ont pas exactement la même conformation spatiale d'une image à l'autre, ce qui module l'absorption locale au cours du temps. Tout comme l'imagerie photoacoustique standard, il est possible de montrer (Voir par exemple le document intitulé « Photoacoustic fluctuation imaging: theory and application to blood flow imaging », par Vilov S., Godefroy G., Arnal B., & Bossy E. ; Optica, 7(11), 1495-1505 (2020)) que dans des conditions idéales (sans bruit et fluctuations parasites, à hématocrite constante), l'image photoacoustique de fluctuation est proportionnelle au produit l'absorption optique locale µ_{λ}(r) multiplié par la fluence de l'impulsion laser Φ_{λ}(r) soit : $σ \left[{A}_{k , λ}^{ideal}\right] \left(r\right) = A . {ϕ}_{λ} \left(r\right) . {μ}_{λ} \left(r\right)$ où r est un vecteur identifiant une position spatiale dans une image en trois dimensions (3D) acquise, A est une constante qui ne dépend pas ni de λ ni de r si l'on peut considérer une invariance spatiale de la fonction d'étalement de point de l'imageur ultrasonore. Si la fonction d'étalement de point varie dans l'espace, alors la dépendance de A en fonction de r peut être prise en compte et corrigée.

Il est possible de déterminer, pour chaque longueur d'onde parmi une pluralité de longueurs d'onde, une image de fluctuations. Cette image de fluctuations est représentative des fluctuations temporelles dues au tissu biologique (par exemple, due au flux sanguin) mais est affectée par la fluctuation pulse à pulse du laser et par le bruit électronique des capteurs d'ondes ultrasonores. En effet, hormis les fluctuations du tissu biologique, il existe d'autres sources de fluctuations parasites: les fluctuations de l'énergie des impulsions du laser et le bruit électronique produit notamment par les capteurs d'ondes ultrasonores du système d'imagerie photoacoustique multispectrale. Il est ainsi nécessaire d'effectuer un traitement spécifique pour que l'image de fluctuations soit représentative uniquement (ou principalement) des fluctuations d'intérêt : ici, les fluctuations d'absorption dues à l'échantillon biologique. L'image de fluctuations qui est obtenue par la méthode décrite dans ce document est en fait proportionnelle à l'absorption des chromophores de l'échantillon biologique responsables des fluctuations d'absorption. Une telle image sera désignée par « image de fluctuations d'absorption ».

Ainsi, en raison des fluctuations de l'énergie des impulsions du laser, l'image de fluctuations, même si elle est obtenue par soustraction de l'image photoacoustique moyenne, comprend toujours un terme résiduel correspondant à l'image photoacoustique moyenne multipliée par la variance de l'énergie d'impulsion. Un filtrage spatio-temporel multispectral par décomposition en valeurs singulières (SVD, Singular Value Decomposition) permet d'éliminer efficacement ce terme résiduel de part sa signature spatio-temporelle spécifique. De manière générale, en supprimant les composantes de l'image correspondant aux premières valeurs singulières, on supprime non seulement les fluctuations de l'énergie des impulsions du laser, les éventuels mouvements (par ex. physiologiques) se produisant dans l'échantillon biologique, mais également la contribution due à l'élément absorbeur moyen dans l'image et on ne garde que les parties de l'image correspondant à des fluctuations temporelles d'intérêt. En outre, il apparaît que la SVD présente une meilleure performance lorsqu'elle est réalisée sur un ensemble d'images multispectrales que lorsqu'elle est appliquée sur des images ayant une même longueur d'onde.

On génère ensuite des images de fluctuations filtrées pour chaque longueur d'onde, à partir d'une succession temporelle d'images filtrées obtenues à l'issue du filtrage spatio-temporel multispectral. Dans l'image de fluctuations filtrée à une longueur d'onde donnée, un pixel de coordonnée r dans l'image de fluctuations étant égal à l'écart type de la distribution des valeurs de pixels de même coordonnée r dans les images filtrées obtenues pour cette longueur d'onde.

Il est possible en outre d'effectuer une correction des images de fluctuations filtrées obtenues pour chaque longueur d'onde pour ne conserver que les fluctuations d'intérêt et obtenir une image des fluctuations d'intérêt ou image de variance d'intérêt correspondante.

Un premier type de correction consiste à corriger des fluctuations dues au bruit électronique des capteurs d'ondes ultrasonores. Cette correction permet à l'image de fluctuations de devenir proportionnelle à Φ_{λ}(r)µ_{λ}(r). Ce bruit étant additif dans l'espace des variances, cette étape peut consister à estimer le bruit de fond dans l'espace image résultant du bruit électronique capté par les capteurs d'ondes ultrasonores et à le soustraire à chaque pixel de l'image de variance pour obtenir une image de variance corrigée dont la racine carrée, l'image de fluctuations corrigée est représentative à chaque pixel uniquement de la fluctuation due au tissu biologique considéré dans le voxel correspondant de l'échantillon (dans l'exemple, par le flux sanguin), pondéré par la distribution de fluence. Cette image de fluctuations corrigée est ainsi proportionnelle en chaque point d'image à l'énergie absorbée en un point de l'espace correspondant au point d'image concerné.

Un deuxième type de correction consiste à normaliser par une fonction de fluence l'image de fluctuations corrigée obtenue à l'issue de la correction des fluctuations dues au bruit électronique des capteurs d'ondes ultrasonores.

L'image de variance brute, non filtrée obtenue à partir des images brutes, non filtrées par SVD, pour une longueur d'onde donnée, correspond en chaque pixel à la somme de la variance représentant les fluctuations induites dans le voxel correspondant de l'échantillon (dans l'exemple, les fluctuations induites par le flux sanguin), pondérée par la fluence d'impulsion, et de la variance du bruit électronique des capteurs d'ondes ultrasonores dans l'espace image.

Sur le plan mathématique, on peut montrer que ${σ}^{2} \left[{A}_{i , λ}\left(r\right)\right] = {var}_{i} \left[{A}_{i , λ}\left(r\right)\right] = {ϕ}_{λ}^{2} \left(r\right) \left(1+{σ}_{ϕ , λ}^{2}\right) {σ}_{λ , flow}^{2} \left(r\right) + {σ}_{ϕ , λ}^{2} {\left|{m}_{λ}\left(r\right)\right|}^{2} + {σ}_{b}^{2}$ où ${σ}^{2} \left[{A}_{i , λ}\left(r\right)\right]$ désigne la valeur du pixel de coordonnées r dans l'image de variance brute obtenue à partir des images brutes acquises (non filtrées par SVD) pour la longueur d'onde λ calculée sur les réalisations indicées par i, ces réalisations correspondant à la succession temporelles des images acquises pour la longueur d'onde λ; ${ϕ}_{λ}^{2} \left(r\right)$ désigne la fluence moyenne au carré du laser pour la longueur d'onde λ au pixel de coordonnées r ; ${σ}_{ϕ , λ}^{2}$ désigne la variance de la fluctuation relative d'énergie pulse à pulse du laser pour la longueur d'onde λ (désignée par « pulse energy fluctuation », PEF, dans la terminologie anglo-saxonne) ; ${\left|{m}_{λ}\left(r\right)\right|}^{2}$ est le module au carré de la valeur du pixel de coordonnées r dans l'image moyenne *m_{λ}* calculée comme la moyenne des images A_{i,λ}(r) acquises à la longueur d'onde λ; ${σ}_{λ , flow}^{2} \left(r\right)$ désigne la valeur du pixel de coordonnées r dans l'image de la variance d'intérêt et représente les fluctuations temporelles dues au tissu biologique dans un voxel donné correspondant au pixel de coordonnée r, normalisé par ${ϕ}_{λ}^{2} \left(r\right)$ ${σ}_{b}^{2}$ désigne la variance du bruit électronique dû aux capteurs d'ondes ultrasonores dans l'image.

On voit que le terme PEF influence deux termes : il biaise le coefficient sur l'image de variance et il est en préfacteur de l'image moyenne. Comme l'image moyenne est d'un à deux ordres de grandeur supérieure à la fluctuation due au tissu biologique, une correction est nécessaire pour retrouver l'image de variance d'intérêt même si le PEF est faible.

La surveillance du PEF à l'aide d'une photodiode en sortie du laser peut également être une méthode de correction insuffisante en raison des fluctuations spatiales du faisceau laser que la photodiode ne permet pas de mesurer. La méthode SVD permet d'éliminer les effets du PEF pour obtenir des images filtrées complètes. L'élimination des premières valeurs singulières lors du filtrage spatio-temporel multispectral par la SVD a pour effet de supprimer les contributions de l'absorbeur moyen soumis à la PEF. Le filtrage SVD modifie aussi le niveau de fond de l'image de variance. De sorte que, après filtrage spatio-temporel multispectral par la SVD on obtient une image de variance filtrée, correspondant à l'image de fluctuations filtrée, telle que : ${σ}^{2} \left[{A}_{i , λ}^{SVD}\left(r\right)\right] = {ϕ}_{λ}^{2} \left(r\right) \left(1+{σ}_{ϕ , λ}^{2}\right) {σ}_{λ , flow}^{2} \left(r\right) + {σ}_{b , SVD}^{2}$ où ${σ}_{b , SVD}^{2}$ désigne la variance du bruit électronique résiduel après filtrage par SVD ; ${σ}_{ϕ , λ}^{2}$ désigne la variance de la fluctuation relative (variance normalisée par le carré de la valeur moyenne) des énergie d'impulsions de laser à la longueur d'onde λ.

La fluctuation relative des impulsions de laser est généralement de quelques pourcents et peut être négligée. En effet, ${σ}_{ϕ , λ} ∼ {10}^{- 2}$ et ${σ}_{ϕ , λ}^{2} ∼ {10}^{- 4}$

On peut donc écrire ${σ}^{2} \left[{A}_{i , λ}^{SVD}\left(r\right)\right] = {ϕ}_{λ}^{2} \left(r\right) {σ}_{λ , flow}^{2} \left(r\right) + {σ}_{b , SVD}^{2}$

Il en découle : $σ \left[{A}_{k , λ}^{ideal}\right] \left(r\right) = {ϕ}_{λ} \left(r\right) {σ}_{λ , flow} \left(r\right) = \sqrt{{σ}^{2} \left[{A}_{i , λ}^{SVD}\left(r\right)\right] - {σ}_{b , SVD}^{2}}$

On voit ainsi qu'on peut obtenir une image de fluctuations d'absorption σ_{λ,flow}(r) représentatives des fluctuations d'absorption du tissu biologique en appliquant deux corrections successivement. La première correction consiste à soustraire en chaque pixel de l'image de variance filtrée, la variance du bruit électronique résiduel après filtrage par SVD pour obtenir une image de variance corrigée puis une image de fluctuation corrigée correspondante en prenant la racine carrée.

La deuxième correction consiste à normaliser l'image de fluctuation corrigée par la fluence de l'impulsion laser Φ_{λ}(r), par division de chaque pixel par Φ_{λ}(r) de sorte à obtenir une image de fluctuation corrigée et normalisée, appelée image de fluctuations d'absorption σ_{λ,flow}(r) ou image de fluctuations d'intérêt. Ces deux corrections sont appliquées pour chaque longueur d'onde considérée.

Les images de fluctuations d'absorption σ_{λ,flow}(r) ainsi obtenues pour chaque longueur d'onde d'excitation sont représentatives uniquement (ou au moins, principalement) des fluctuations d'absorption induites par le tissu biologique. Dans le cas où les fluctuations d'absorption dans le tissu biologique sont principalement celles dues au flux sanguin, σ_{λ,flow}(r) représente les fluctuations d'absorption dues au flux sanguin.

Une approche par imagerie de fluctuations permet ainsi de résoudre les problèmes de visibilité mentionnés en introduction, ainsi que d'améliorer le contraste, au prix de l'acquisition d'une série d'images. On peut par exemple obtenir des informations sur les fluctuations à l'intérieur des vaisseaux de grande taille et des informations sur les vaisseaux orientés verticalement.

Ce procédé est utile notamment dans le cas d'acquisition d'images au moyen de dispositifs d'acquisition fixes tenus à la main (appelé « handheld devices » ou « single-sided devices ») ou tenu par une pièce mécanique, qui sont placés sur la peau du sujet pour imager une région donnée, sans nécessiter de déplacement lors de l'acquisition. Dans de tels dispositifs d'acquisition, les capteurs d'ondes acoustiques sont par exemple des capteurs piézo-électriques à visibilité limitée (bande passante et spectre angulaire de réception limités).

En outre, une analyse quantitative du taux d'oxygénation sanguin est possible. En effet, dans le cas de l'application à l'imagerie de l'oxygénation des vaisseaux sanguins, à partir des différentes images de fluctuations d'absorption obtenues pour différentes longueurs d'onde, on peut déterminer des cartes d'oxygénation 2D ou 3D par inversion d'un modèle liant les cartes de fluctuation dues au flux sanguin aux différentes longueurs d'onde normalisées par la fluence, au taux d'oxygénation. Ce qui permet une quantification des spectres d'absorption optique des vaisseaux sanguins. Plus précisément, en utilisant des ondes lumineuses d'excitation des tissus à plusieurs longueurs d'onde, on peut déterminer la quantité relative ou absolue d'oxyhémoglobine et de desoxyhémoglobine à partir des différences de profil d'absorption obtenues selon la longueur d'onde.

Pour obtenir une carte d'oxygénation, on peut partir soit des images de fluctuations corrigées et normalisées par la fluence incidente dans le cas où il n'y a pas de coloration spectrale due au tissu, soit des images de fluctuations d'absorption qui sont des images de fluctuations corrigées et normalisées par la fluence spatiale au sein de l'échantillon. Dans le premier cas, en chaque point on est proportionnel aux images de fluctuations d'absorption mais on ne corrige pas l'atténuation de la lumière en profondeur (qui est supposée être la même pour chaque longueur d'onde lorsqu'il n'y a pas de coloration spectrale due au tissu).

On obtient ainsi une imagerie quantitative du taux d'oxygénation des vaisseaux sanguins. L'image de fluctuations d'absorption est spécifique aux écoulements sanguins, apportant une spécificité dans les évaluations quantitatives, notamment lorsqu'il s'agit de vaisseaux sous-résolus entourés d'autres absorbeurs et évite la complexité d'un démélange spectral (« spectral unmixing »). Cette méthode peut être notamment appliquée à l'imagerie de tumeurs, à l'imagerie du cerveau et à l'imagerie vasculaire. Comme dit plus haut, l'image de fluctuation a un meilleur contraste que l'image conventionnelle, et n'est pas affectée par les artefacts de visibilité présents en imagerie conventionnelle. Le taux d'oxygénation est donc obtenu sur une image bien plus riche que l'image conventionnelle.

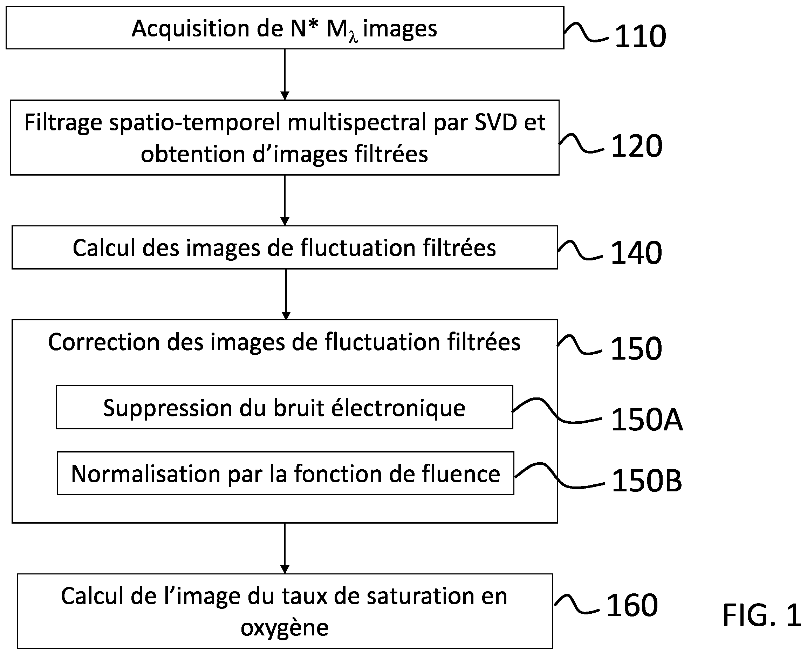

La fig. 1 montre un organigramme simplifié d'un procédé de traitement d'images acquises par un système d'imagerie photoacoustique multispectrale.

Dans une étape 110, une succession temporelle d'images est acquise par un système d'imagerie photoacoustique pour M_{λ} longueurs d'ondes, soit N images par longueur d'onde et N* M_{λ} images au total. Les images acquises sont notées *A_{i,j}* où i est un entier compris entre 1 et N représente l'index de l'image acquise pour une longueur d'onde donnée ; j est un entier compris entre 1 et M_{λ} et représente l'index identifiant la longueur d'onde concernée. Un pixel d'une image *A_{i,j}* est noté *A_{i,j}*(*r*) où r est la coordonnée d'un pixel de cette image.

La cadence d'acquisition est fixe, par exemple de 100 Hz. Typiquement on utilise par exemple M_{λ}=6 longueurs d'onde et N=250 images par longueur d'onde. Plus généralement on peut prendre N compris entre 10 et 100000, M_{λ} compris entre 2 et 100, et la fréquence d'acquisition peut être comprise entre 10 et 100000 Hz.

L'acquisition peut se faire par exemple en faisant varier la longueur d'onde d'une image à la suivante de manière cyclique : une image avec une première longueur d'onde λ₁, puis une image avec une deuxième longueur d'onde λ₂, et ainsi de suite jusqu'à la dernière longueur d'onde λ_{M}, puis en répétant N fois le même cycle d'acquisition avec les longueurs d'onde λ₁ à λ_{M}. De cette manière la période entre deux acquisitions à une même longueur d'onde est fixe et identique quelle que soit la longueur d'onde. Dans le cas où seulement deux longueurs d'onde sont utilisées, on alterne la première longueur d'onde λ₁ avec la deuxième longueur d'onde λ₂.

Les images acquises *Aᵢⱼ* font l'objet d'un post-traitement comprenant les étapes 120 à 150 définies ci-dessous.

Dans une étape 120, un filtrage spatio-temporel multispectral par décomposition en valeurs singulières (Singular Decomposition Value, SVD) est appliqué à l'ensemble des N*M_{λ} images et on obtient à l'issue de l'étape N* M_{λ} images filtrées notées ${A}_{i , j}^{SVD}$

Ce filtrage spatio-temporel multispectral par SVD permet de supprimer efficacement dans l'image de variance les composantes de l'absorbeur moyen, notamment le terme ${σ}_{ϕ , λ}^{2} {\left|{m}_{λ}\left(r\right)\right|}^{2}$ résultant des fluctuations de l'énergie d'impulsion du laser et de l'image moyenne, qui par son amplitude, masque les fluctuations d'intérêt. Le fait d'utiliser un filtrage par SVD pour plusieurs longueurs d'onde rend la SVD statistiquement plus performante et permet d'obtenir des composantes représentant des fluctuations du tissu en réponse à plusieurs longueurs d'onde d'excitation. Si on devait utiliser le filtrage spatio-temporel par SVD sur des images acquises pour une seule longueur d'onde, le choix des bornes de filtrage SVD varierait d'une longueur d'onde à l'autre, ce qui alourdirait la procédure.

Dans une étape 140, pour chaque longueur d'onde séparément, on détermine une image de fluctuations filtrée (respectivement, une image de variance filtrée): l'écart type (respectivement la variance) de la distribution des valeurs de pixel de même coordonnée r dans les N images filtrées obtenues à l'étape 120 pour cette longueur d'onde est calculée pour obtenir le pixel de coordonnée r dans l'image de fluctuations filtrée(respectivement dans une image de variance filtrée). On obtient ainsi pour chaque longueur d'onde j, à l'issue de l'étape 140, M_{λ} images de variance notées ${σ}_{j}^{2} \left[{A}_{i , j}^{SVD}\right]$ ou plus simplement ${σ}_{j}^{2}$

Avec cette notation, ${σ}_{j}^{2} \left[{A}_{i , j}^{SVD}\right]$ où ${σ}_{j}^{2} \left(r\right)$ désigne la variance de la distribution des valeurs ${σ}_{j}^{2} \left[{A}_{i , j}^{SVD}\right]$ au pixel de coordonnée r calculée sur les réalisations i= 1 à N pour la longueur d'onde j.

Dans une étape 150, une correction de chaque image de fluctuations filtrée est effectuée de sorte à obtenir une image de fluctuations d'absorption représentative, uniquement ou essentiellement, des fluctuations d'absorption de l'échantillon biologique. La correction de l'image de fluctuations filtrée comprend ainsi une suppression de fluctuations autres que les fluctuations d'intérêt dues à l'échantillon, de sorte à obtenir une image de fluctuations d'absorption représentant des fluctuations d'absorption dues à l'échantillon. Deux types de corrections sont utilisables en combinaison ou isolément.

Un premier type de correction (étape 150A) consiste en une correction de fluctuations dues au bruit électronique de capteurs d'ondes ultrasonores par soustraction, à chaque pixel de coordonnée r dans l'image de variance filtrée, de la variance du bruit électronique résiduel après SVD, la variance du bruit électronique résiduel après SVD étant noté : ${σ}_{b , SVD}^{2}$ soit ${ϕ}_{λ}^{2} \left(r\right) {σ}_{λ , flow}^{2} \left(r\right) = {σ}^{2} \left[{A}_{i , λ}^{SVD}\left(r\right)\right] - {σ}_{b , SVD}^{2}$

La variance du bruit électronique résiduel des capteurs d'acquisition est estimée puis la variance ${σ}_{b , SVD}^{2}$ du bruit électronique résiduel est soustraite en chaque pixel de l'image de variance filtrée obtenue à l'étape 140, de sorte à obtenir une image de variance corrigée pour une longueur d'onde donnée. La variance du bruit électronique résiduel après SVD peut correspondre à la variance du bruit électronique avant SVD si la borne b du filtrage SVD est égale au nombre total d'images. Une image de fluctuations corrigée correspondante peut être obtenue en calculant la racine carrée de l'image de variance corrigée.

Cette image de fluctuations corrigée peut ensuite être pondérée par la fluence à la longueur d'onde considérée. Cette image de fluctuations corrigée est proportionnelle en chaque point d'image à l'énergie absorbée en un point de l'espace correspondant au point d'image concerné.

Le bruit électronique produit par les capteurs d'ondes ultrasonores peut être estimé en fonction d'une variance du bruit électronique produit dans les signaux photoacoustiques acquis en l'absence d'échantillon, la variance étant corrigée par une quantité de bruit éliminée par le filtrage spatio-temporel multispectral par décomposition en valeurs singulières, cette quantité de bruit pouvant être estimée sur la base des valeurs singulières correspondant aux composantes de plus basse énergie supprimées par le filtrage spatio-temporel multispectral par décomposition en valeurs singulières.

Un deuxième type de correction (étape 150B) consiste en une normalisation par une fonction de la fluence d'impulsions laser à la longueur d'onde considérée. Une image de variance normalisée par la fluence peut être obtenue pour chaque longueur d'onde par normalisation par la fonction spatiale de fluence au carré ${ϕ}_{λ}^{2} \left(r\right)$ pour chaque longueur d'onde j=1 à M_{λ} de sorte à obtenir une image de variance normalisée ${σ}_{λ , flow}^{2} \left(r\right)$ représentative uniquement des fluctuations d'absorption produites par l'échantillon (par exemple, par le flux sanguin), de sorte que ${σ}_{λ , flow}^{2} \left(r\right)$ représente la valeur du pixel de l'image de variance normalisée obtenue pour la longueur d'onde λ au pixel de coordonnée r.

La normalisation par la fluence peut être effectuée sur l'image de variance corrigée en utilisant la fonction de fluence au carré Φ²_{λ}(r) de sorte à obtenir une image de variance normalisée puis une image de fluctuations normalisée correspondante par calcul de la racine carrée de chaque pixel. Alternativement, et de manière équivalente, la normalisation par la fluence peut être effectuée sur l'image de fluctuations correspondant à l'image de variance corrigée: ${ϕ}_{λ} \left(r\right) {σ}_{λ , flow} \left(r\right) = \sqrt{{σ}^{2} \left[{A}_{i , λ}^{SVD}\left(r\right)\right] - {σ}_{b , SVD}^{2}}$ en calculant la racine carrée de chaque pixel de l'image de variance corrigée obtenue après correction du bruit électronique avant d'effectuer la normalisation par la fluence de l'impulsion laser Φ_{λ}(r).

Suite à cette normalisation, on obtient au final une image de fluctuations normalisée, qui est l'image de fluctuations d'absorption σ_{λ,flow}(r) représentant les fluctuations d'absorption dues à l'échantillon.

Concernant la détermination de la fonction de fluence à utiliser pour la normalisation, différentes méthodes et approximations sont possibles.

Au lieu d'utilisation d'une fonction spatiale de la fluence, on peut utiliser une fluence moyenne (indépendante du point de l'espace) à une longueur d'onde donnée. La fluence moyenne à une longueur d'onde peut être déterminée par exemple sur la base de mesures d'énergie des impulsions d'excitations obtenues au moyen d'une photodiode placée en sortie du laser au moment où une impulsion d'excitation à une longueur d'onde donnée est envoyée vers l'échantillon. Les intensités des signaux électriques produits par la photodiode sont converties en estimation de la fluence à l'aide de coefficients de calibration, l'estimation de la fluence étant utilisée pour calculer une fluence moyenne et effectuer ensuite la correction par normalisation.

On peut définir la fonction spatiale de fluence de la manière suivante : ${ϕ}_{λ} \left(r\right) = {ϕ}_{λ} \left(0\right) {ξ}_{λ} \left(r\right)$

Où ${ϕ}_{λ} \left(0\right)$ est la fluence mesurée à la surface de l'échantillon qui peut être obtenue par une photodiode calibrée et ${ξ}_{λ} \left(r\right)$ représente la variation spatiale relative de la fluence.

Dans le cas général, une estimation de la distribution spatiale de fluence peut être obtenue par des modélisations ou des mesures.

Dans certains cas, on peut approximer qu'il n'y a pas de coloration spectrale dû au tissu. Dans ce cas, on peut écrire ${ξ}_{λ} \left(r\right) = ξ \left(r\right)$

Dans ce cas, une estimation d'oxygénation peut être réalisée simplement à partir de la normalisation par la fluence de la photodiode car la même constante $ξ \left(r\right)$ est utilisable pour l'ensemble des longueurs d'onde.

Lorsque la fluence est uniforme spatialement (hypothèse des embryons de poulet), on peut de même estimer la fluence directement à partir des signaux produits par la photodiode, soit: ${ϕ}_{λ} \left(r\right) = {ϕ}_{λ} \left(0\right)$

Dans une étape 160, on détermine une image α(r) du taux de saturation en oxygène à partir d'au moins deux images de fluctuations d'absorption σ_{λ,flow} obtenues à l'issue de l'étape 150 pour au moins deux longueurs d'onde λ. En alternative l'étape 160 peut être également être exécutée à partir d'au moins deux images de fluctuations corrigées σ_{λ} (avec les corrections selon l'étape 150A mais sans les corrections selon l'étape 150B) obtenues en calculant la racine carrée de chaque pixel des images de variance correspondantes obtenues à l'issue de l'étape 140.

On décrit le cas d'exemple où l'étape 160 est exécutée à partir des images de fluctuations d'absorption σ_{λ,flow} obtenues à l'issue de l'étape 150 (les mêmes calculs étant utilisables en partant des images de fluctuations corrigées n'ayant pas subi les corrections selon l'étape 150B). On utilise pour cela, pour chaque pixel de coordonnées r, un modèle exprimant la relation entre la valeur ${σ}_{λ , flow} \left(r\right)$ au pixel r de l'image de fluctuations d'absorption et deux paramètres, K(r), incluant la concentration totale en hémoglobine et un coefficient de sensibilité de l'électronique de réception et le taux de saturation en oxygène α(r).

Le modèle consiste par exemple en une équation à deux inconnues K(r) et α(r) donnant la fluctuation ${σ}_{λ , flow} \left(r\right)$ en fonction de K(r) et α(r), où r est la coordonnée d'un pixel d'image. On peut donc déterminer pour chaque pixel de coordonnée r le taux de saturation en oxygène α(r) et générer ainsi une image α comprenant pour chaque pixel une information quantitative de la saturation en oxygène (comprise entre 0% et 100%). On peut de même générer une image K comprenant pour chaque pixel une information quantitative proportionnelle à la concentration totale en hémoglobine K(r), aussi appelée « blood volume ».

Dans un mode de réalisation, le modèle est un modèle analytique basé sur l'équation suivante : ${σ}_{λ , flow} \left(r\right) = K \left(r\right) \left(α\left(r\right){μ}_{λ , {HbO}_{2}}+\left(1-α\left(r\right)\right){μ}_{λ , Hbb}\right)$

Dans laquelle ${μ}_{λ , {HbO}_{2}}$ désigne le coefficient d'absorption de l'oxyhémoglobine à la longueur d'onde λ et ${μ}_{λ , Hbb}$ désigne le coefficient d'absorption de la desoxy-hémoglobine à la longueur d'onde λ ;
K(r) désigne un pré-facteur qui dépend de la position r mais pas de la longueur d'onde λ.

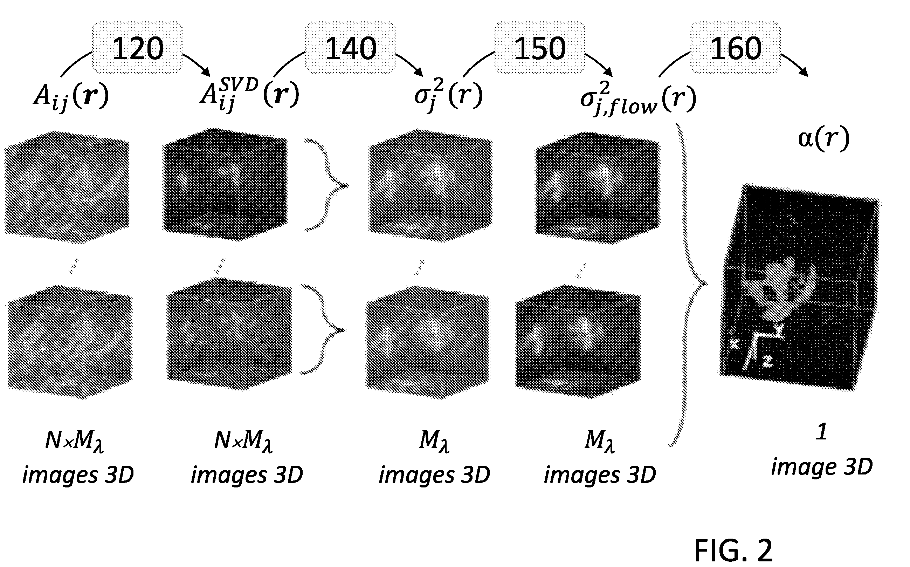

La fig. 2 illustre des aspects des étapes 120 à 160 sur la base d'images d'exemples obtenues à chaque étape.

Les images acquises A_{i,j} obtenues en entrée sont dans cet exemple des images en trois dimensions (3D). On a donc N* M_{λ} images 3D acquises notées ${A}_{1 , 1}$ à ${A}_{N , {M}_{λ}}$

A l'issue de l'étape 120 (SVD), on a N* M_{λ} images 3D filtrées notées ${A}_{1 , 1}^{SVD}$ à ${A}_{N , {M}_{λ}}^{SVD}$

A l'issue de l'étape 140 (calcul de l'image de la variance), on a M_{λ} images 3D de fluctuations notées ${σ}_{{λ}_{1}}^{2}$ à ${σ}_{{λ}_{M}}^{2}$ pour des longueurs d'onde j= λ₁ à λ_{M}. A l'issue de l'étape 150 (correction de bruit et/ou normalisation par la fluence), on a M_{λ} images 3D de variance représentant les fluctuations d'absorption notées ${σ}_{1 , flow}^{2} \left(r\right)$ à ${σ}_{{M}_{λ} , flow}^{2} \left(r\right)$ pour des longueurs d'onde j= λ₁ à λ_{M}. Enfin à l'issue de l'étape 160 (calcul de la saturation en oxygène), on obtient une image 3D du taux de saturation en oxygène notée α(r).

La fig. 2 permet d'observer sur un cas d'exemple les effets des différents traitements sur les différentes images 3D. En comparant les images acquises aux images correspondantes filtrées après SVD suite à l'étape 120, on observe que seuls les points des images correspondant à des fluctuations de flux sont conservés. En comparant les images filtrées après SVD suite à l'étape 120 aux images de fluctuations filtrées obtenues suite à l'étape 140, on observe que certaines structures apparaissent dans les images de fluctuations filtrées. En comparant les images de fluctuations filtrées obtenues suite à l'étape 140 aux images de fluctuations d'absorption obtenues suite à l'étape 150, on observe que le niveau de fond est ramené à zéro. Enfin, on observe que suite à l'étape 160, on obtient une image volumique du taux de saturation en oxygène.

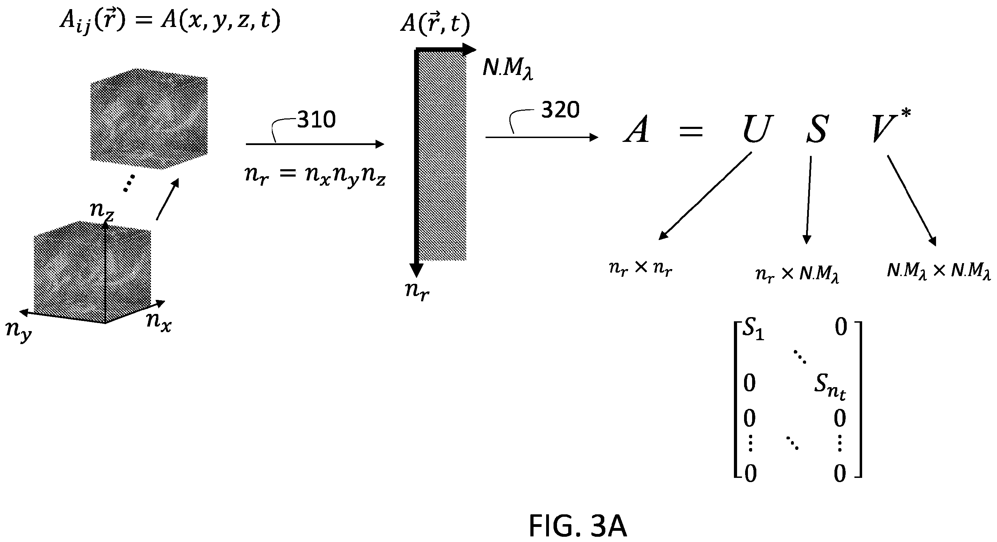

Les Figs. 3A-3B illustrent une méthode de filtrage spatio-temporel multispectral par SVD utilisable pour l'étape 120 selon un exemple de réalisation.

Selon ce qui est illustré par la fig. 3A, une succession temporelle d'images multispectrales (1000) est acquise par un système d'imagerie photoacoustique pour M_{λ} longueurs d'ondes, soit N images par longueur d'onde et N* M_{λ} images au total. Les images acquises A_{i,j} obtenues en entrée sont des images en 3D supposées de taille identique n_{X} * n_{Y} * n_{Z} : le nombre de pixels est n_{X} selon un premier axe X d'un espace cartésien 3D, n_{Y} selon un deuxième axe Y et n_{Z} selon un troisième axe Z.

A partir de cette succession temporelle d'images multispectrales, on forme lors d'une étape 310, une matrice de Casorati (301) en deux dimensions (2D) représentant respectivement l'espace et le temps, notée *A*(*r,t*), où t désigne un index temporel et r un scalaire correspondant à une position d'un pixel dans l'espace 3D dans lequel les N* M_{λ} images sont définies. La matrice de Casorati (301) est de taille n_{R} * (N* M_{λ}), avec n_{R} = n_{X} * n_{Y} * n_{Z}. La matrice de Casorati (301) contient les pixels des N* M_{λ} images. La valeur t de l'index temporel peut ainsi varier de t=1 à N* M_{λ} de sorte que A(r,1)= A_{1,1}(r) est la première image de la succession temporelle pour une première longueur d'onde, A(r,2)= A_{1,2}(r) est la deuxième image de la succession temporelle pour une deuxième longueur d'onde, et ainsi de suite. Il est à noter que dans la notation A(r,1), r désigne un scalaire tandis que dans la notation A_{i,j}(r), r désigne un vecteur de coordonnées (x,y,z) dans un espace à trois dimensions. On peut calculer le scalaire r à partir des coordonnées du vecteur r=(x,y,z) et de la taille n_{X} * n_{Y} * n_{Z} des images acquises. L'utilisation de la matrice de Casorati (301) permet de réaliser l'analyse des fluctuations dans le domaine spatio-temporel.

Lors d'une étape 320, la matrice de Casorati (301) est décomposée selon une méthode de décomposition en valeurs singulières : A = U S V* où U est une matrice 2D de taille n_{R}* n_{R} comprenant les vecteurs singuliers spatiaux, S est la matrice 2D des valeurs singulières de taille n_{R}* (N* M_{λ}) dont les coefficients diagonaux Sₖ sont des réels positifs et V* est la matrice transposée conjuguée de V, V étant la matrice des vecteurs singuliers spatiaux de taille (N* M_{λ})*(N* M_{λ}). Un exemple de matrice S (302) est représenté à la fig. 3A. Une convention courante est de ranger les valeurs Sₖ par ordre de valeurs décroissantes.

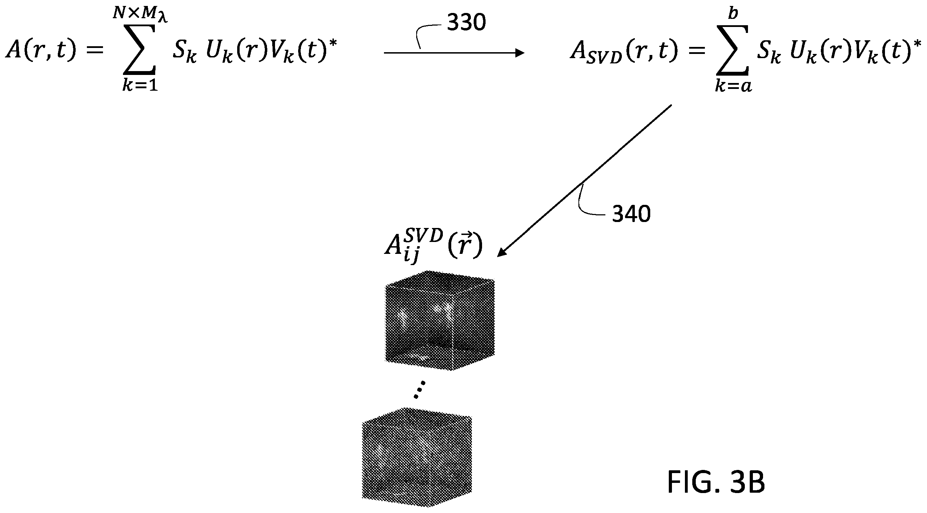

Selon ce qui est illustré par la fig. 3B, sur la base de la décomposition en valeurs singulières, la matrice *A*(*r,t*) peut s'exprimer sous la forme d'une somme pondérée de matrices dont les coefficients de pondération sont les coefficients diagonaux Sₖ de la matrice 2D des valeurs singulières : $A \left(r, t\right) = {\sum }_{k = 1}^{N \times {M}_{λ}} {S}_{k} {U}_{k} \left(r\right) {V}_{k} \left(t\right) *$

Dans cette somme, chaque matrice Uₖ(r)Vₖ(t)* définit une composante correspondant à la valeur singulière Sₖ. Afin d'effectuer un filtrage, seules certaines composantes sont conservées et donc seules certaines matrices de cette somme pondérée sont conservées. Lorsque les valeurs Sₖ sont rangées par ordre de valeurs décroissantes, il s'agit donc de sélectionner lors d'une étape 330 des valeurs d'index minimal ***a*** et d'index maximal b de l'index k de sorte que la matrice obtenue après filtrage spatio-temporel multispectral est ${A}_{SVD} \left(r, t\right) = {\sum }_{k = a}^{b} {S}_{k} {U}_{k} \left(r\right) {V}_{k} \left(t\right) *$

Le choix de la valeur de l'index minimal ***a*** est important en ce qu'il détermine l'efficacité et la pertinence du filtrage en déterminant quelles sont les composantes de plus grande énergie qui seront éliminées par filtrage. Le choix de la valeur de l'index maximal b a un impact moins sensible sur le filtrage car il concerne les composantes de plus faible énergie qui incluent du bruit pur et potentiellement de l'information noyée dedans et peut être choisi par exemple égale à a+100 ou être comprise entre a+1 et N* M_{λ} .

Après suppression des composantes correspondantes aux valeurs singulières de plus haute énergie, on effectue lors d'une étape 340 une transformation inverse à celle effectuée de l'étape 310 à partir de la matrice A_{SVD}(r,t) pour obtenir une succession temporelle d'images filtrées notées ${A}_{i , j}^{SVD} .$

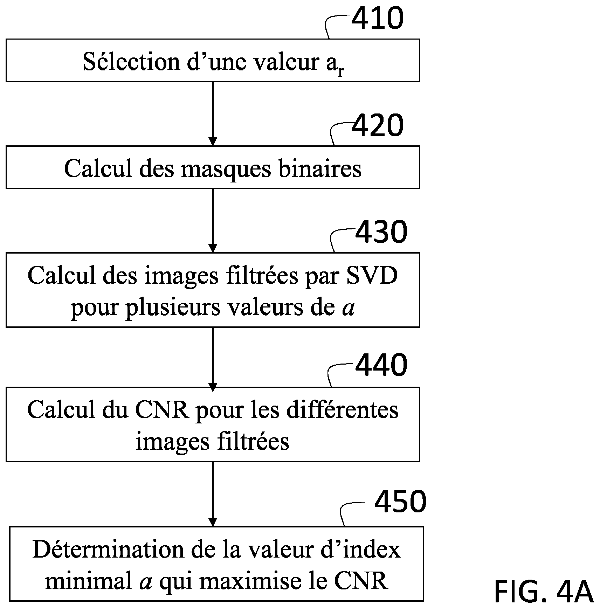

Les Figs. 4A-4B illustrent des aspects d'une méthode de sélection de composantes à supprimer lors du filtrage spatio-temporel multispectral par SVD effectué à l'étape 120.

La fig. 4A montre un organigramme simplifié d'un procédé de sélection des composantes à éliminer lors du filtrage spatio-temporel multispectral par SVD effectué lors de l'étape 120, notamment la sélection de la valeur de l'index minimal ***a*** (appelée aussi borne inférieure) identifiant quelles composantes seront éliminées par filtrage.

Le choix de l'index minimal ***a*** peut être délicat en ce que, en prenant des valeurs d'index minimal ***a*** trop grandes, on risque de supprimer des parties des objets représentés dans une image. La méthode proposée ici permet de sélectionner une valeur d'index minimal ***a*** de manière automatisée et sans risquer de supprimer des parties des objets représentés dans une image.

Le procédé est basé sur une estimation d'un rapport contraste à bruit (Contrast-to-Noise Ratio, CNR) estimé pour des images filtrées par SVD (sans correction de bruit selon l'étape 150A ni normalisation par la fluence selon l'étape 150B) pour plusieurs valeurs de l'index a et on détermine quelle est la valeur de l'index minimal ***a*** qui maxime ce rapport contraste à bruit.

L'évaluation du rapport contraste à bruit est effectuée sur la base d'une comparaison entre des images filtrées par SVD et masquées pour extraire les structures qui y apparaissent. Ce masque est obtenu à partir des étapes 410 et 420.

A l'étape 410, on sélectionne arbitrairement une valeur de référence aᵣ de l'index minimal ***a***. Par exemple aᵣ= 0.03*N*M_{λ}=0.03*10*100=30. Cette valeur peut être sélectionnée de manière empirique à partir d'une analyse sur quelques échantillons représentatifs.

Puis on sélectionne une image de variance de référence à une seule longueur d'onde notée ${σ}_{{a}_{r} , {j}_{max}}^{2} \left(r\right)$ pour cette valeur de référence aᵣ de l'index minimal ***a*** et pour une longueur d'onde donnée λ=λⱼₘₐₓ, qui est par exemple la longueur d'onde pour laquelle le SNR sur les signaux bruts (« Signal to Noise Ratio », rapport signal à bruit) est le plus élevé. Le calcul de ${σ}_{{a}_{r} , {j}_{max}}^{2} \left(r\right)$ comprend l'exécution des étapes 120 (SVD) et 140 (image de variance filtrée) pour les images acquises pour la longueur d'onde d'indice jₘₐₓ, mais pas l'étape 150 de correction.

A l'étape 420, on calcule 2 images binaires servant de masques binaires :
*M_{σ}* est le masque binaire calculé sur la base de l'image de variance de référence ${σ}_{{a}_{r} , {j}_{max}}^{2} \left(r\right)$
sur la base d'une valeur de seuil ***th_{σ}*;**
*Mₘ* est un masque binaire calculé sur la base de l'image moyenne $\begin{matrix} \\ {\left〈A\right〉}_{jmax}\end{matrix}$
dont chaque pixel est calculé comme la moyenne des pixels ${A}_{i , jmax} \left(r\right)$
sur i, donc sur l'ensemble des images acquises pour au moins une longueur d'onde donnée, choisie par exemple comme étant λ=λⱼₘₐₓ- Une autre valeur de seuil ***thₘ*** est utilisée.

La détermination de la valeur de seuil ***th_{σ}*** permettant d'obtenir le masque binaire ***M_{σ}*** peut être effectuée sur la base de la formule suivante : ${th}_{σ} = {σ}_{b}^{2} + θ \times {std}_{r} \left({σ}_{{a}_{r} {, j}_{max}}^{2}\left(r\right)\right)$ où ${σ}_{b}^{2}$ désigne la variance spatio-temporelle du bruit électronique, déterminé comme par exemple décrit ci-dessus pour l'étape 150A. ${std}_{r} \left({σ}_{{a}_{r} {, j}_{max}}^{2}\left(r\right)\right)$ désigne l'écart type intra-image calculé sur la distribution des pixels de l'image de variance de référence;
θ est un coefficient de pondération égal par exemple à 0.35, obtenu empiriquement en s'assurant sur quelques exemples que le masque ***M_{σ}*** obtenu correspond bien à la structure observable sur l'image ${σ}_{{a}_{r} , {j}_{max}}^{2} \left(r\right)$

La détermination de la valeur de seuil ***thₘ*** permettant d'obtenir le masque binaire ***Mₘ*** peut être effectuée sur la base de la formule suivante : ${th}_{m} = {\left〈{\left〈A\right〉}_{{j}_{max}}\right〉}_{r} + θ \times {std}_{r} \left({\left〈A\right〉}_{{j}_{max}}\right)$ où ${\left〈{\left〈A\right〉}_{{j}_{max}}\right〉}_{r}$ désigne la valeur moyenne des pixels de l'image moyenne notée ${\left〈A\right〉}_{{j}_{max}}$ et ${std}_{r} \left({\left〈A\right〉}_{{j}_{max}}\right)$ désigne l'écart type intra-image calculée sur la distribution des pixels de l'image moyenne ${\left〈A\right〉}_{{j}_{max}}$

Le coefficient θ est le même que celui utilisé pour obtenir le masque ***M_{σ}.***

Un masque binaire sert à distinguer dans une image le fond d'image (« background ») du reste, notamment du ou des objets représentés dans cette image. De manière connue, pour calculer un masque binaire sur la base d'une image et d'une valeur de seuil, on détermine si la valeur du pixel de l'image est supérieure (ou égale) au seuil : dans l'affirmative, la valeur du pixel correspondant dans le masque binaire est égale à 1 et dans la négative la valeur du pixel correspondant dans le masque binaire est égale à 0.

Les deux masques binaires obtenus sont utilisés dans la formule du CNR dont le calcul permet de déterminer l'index minimal ***a*** à utiliser pour le filtrage spatio-temporel multispectral par SVD. Le filtrage est réalisé sur toutes les longueurs d'onde et l'index minimal ***a*** est le même pour toutes les longueurs d'onde.

Dans une étape 430, plusieurs valeurs de l'index minimal ***a*** sont sélectionnées, par exemple parmi un ensemble de valeurs prédéfinies tel que 1 et 100, et on calcule les images de variance à toutes les longueurs d'onde, notée ${σ}_{a , j}^{2} \left(r\right)$ pour chaque valeur de l'index minimal ***a*.**

Dans une étape 440, on calcule une valeur de rapport contraste à bruit CNR(a) pour chaque valeur de l'index minimal ***a*** en utilisant les deux masques binaires.

Dans une étape 450 on sélectionne comme borne inférieure la valeur de l'index minimal ***a*** pour laquelle le rapport contraste à bruit CNR(a) est maximal. On utilise l'image de variance ${σ}_{a , j}^{2} \left(r\right)$ correspondant à cette borne inférieure pour effectuer la suite des traitements (notamment étapes 150 et 160 du procédé décrit par référence à la fig. 1).

Le rapport contraste à bruit (Contrast-to-Noise Ratio, CNR) peut se calculer de différentes manières sur la base d'un masque binaire permettant de distinguer quels pixels de l'image font partie du fond d'image et quels pixels de l'image font partie du ou des objets représentés dans cette image. De manière générale le rapport contraste à bruit est le rapport entre, d'une part, le contraste déterminé comme la différence entre la valeur moyenne des pixels faisant partie du ou des objets et la valeur moyenne des pixels faisant partie du fond d'image vis-à-vis de la fluctuation du fond d'image. Le choix du rapport contraste à bruit comme critère résulte notamment du fait que, pour détecter à l'œil des pathologies dans une image, il faut que son contraste soit supérieur à la fluctuation du fond d'image, et donc que le rapport contraste à bruit soit supérieur à 1.

Selon un exemple de réalisation, le rapport contraste à bruit est calculé pour chaque image de variance à chaque longueur d'onde ${σ}_{a , j}^{2} \left(r\right)$ obtenue pour la valeur de l'index minimal ***a*,** puis moyenné sur les longueurs d'onde, comme suit : $CNR \left(a\right) = \frac{1}{M} {\sum }_{j}^{M} \frac{{\left〈\left({M}_{σ}-\left({M}_{σ}∩{M}_{m}\right)\right).{σ}_{a , j}^{2}\right〉}_{r} - {\left〈\overline{{M}_{σ}}.{σ}_{a , j}^{2}\right〉}_{r}}{{std}_{\overline{r}} \left(\overline{{M}_{σ}}.{σ}_{a , j}^{2}\right)}$ où
M désigne le nombre de longueurs d'onde $∩$
est l'opérateur représentant l'intersection des deux maques binaires, l'intersection correspondant à une fonction « ET » logique effectuée pixel à pixel entre les deux masques ; ${std}_{\vec{r}}$
désigne l'opération d'écart type intra-image calculée sur la distribution des pixels ; $\overline{{M}_{σ}}$
désigne le complémentaire du masque ***M_{σ},*** soit *1- **M_{σ}*** $\overline{{M}_{σ}} . {σ}_{a , j}^{2}$
désigne le terme correspondant aux pixels appartenant au fond de l'image 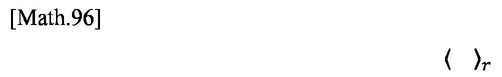
désigne l'opération de moyenne intra-image calculée sur la distribution des pixels Ce type de formule de calcul permet, grâce au terme
d'éliminer dans le calcul du rapport contraste à bruit les structures à la fois présentes dans l'image moyenne et dans l'image de variance. Dans les images acquises par une technique d'imagerie photoacoustique la valeur moyenne de l'image est environ 100 fois plus grande que le reste, ce qui donnerait une valeur de CNR(a) très élevée si l'on ne soustrayait pas le contraste dû à la valeur moyenne de l'image pour les faibles valeurs de l'index minimal ***a*.** Le masque utilisé assure ainsi que le contraste augmente lorsque de nouvelles structures apparaissent. Ainsi pour pouvoir comparer de manière pertinente les valeurs de CNR(a) quelle que soit l'index minimal ***a*,** on utilise cette formule de calcul spécifique.

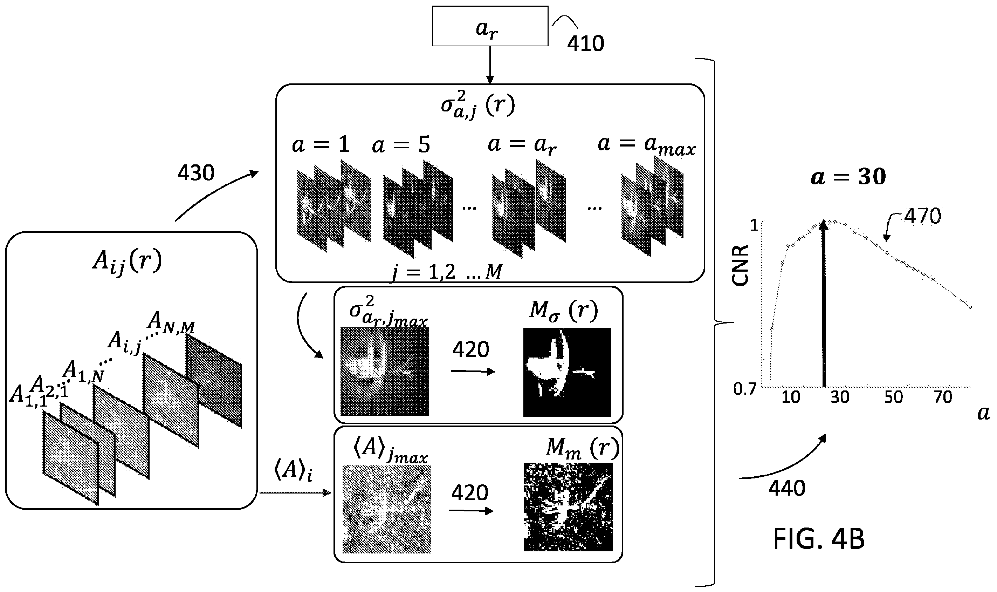

La courbe d'exemple (470) des variations de CNR(a) présentée à la fig. 4B montre que le rapport contraste à bruit CNR(a) présente un maximum pour une valeur de l'index minimal ***a*** comprise entre 25 et 28. Pour un autre exemple, la valeur de l'index minimal ***a*** est comprise entre 33 et 35. En ce qui concerne l'estimation du bruit électronique des capteurs utilisés à l'étape 150A, plusieurs méthodes sont utilisables.

Selon une première méthode d'estimation du bruit, applicable dans le cas d'une image ${σ}_{j}^{2}$ reconstruite, le bruit électronique dans l'image reconstruite peut être déduit d'une mesure de bruit dans l'espace des signaux radiofréquence réels. ${σ}_{b}^{2} = 2 {N}_{trans} {σ}_{b , RF}^{2}$ où ${N}_{trans}$ désigne le nombre de transducteurs utilisés dans la reconstruction ${σ}_{b , RF}^{2}$ désigne la variance du bruit électronique sur les signaux radiofréquence mesurés et est déterminé à partir de signaux bruts obtenus en l'absence d'échantillon.

Le facteur 2 vient ici du fait que l'on utilise des signaux à valeurs complexes pour la reconstruction.

Cette estimation de ${σ}_{b}^{2}$ peut toutefois être corrigée en considérant que le filtrage spatio-temporel multispectral par SVD supprime une quantité de bruit égale à $Δ {σ}_{SVD}^{2} = \frac{1}{{NM}_{λ} {N}_{X} {N}_{Y} {N}_{Z}} {\sum }_{k = b + 1}^{{NM}_{λ}} {S}_{k}^{2}$ où b est l'index maximal de la somme pondérée de matrices définissant ${A}_{SVD} \left(r, t\right)$ N_{X} N_{Y} N_{Z} désigne le nombre de pixels/voxels de l'image reconstruite et
Sk les valeurs singulières obtenues par la SVD.

Il a été observé que les valeurs singulières représentant le bruit présentes dans l'expression de ${σ}_{SVD}^{2}$ sont stationnaires par rapport aux longueurs d'ondes.

Au final, le bruit électronique résiduel après SVD à soustraire à l'étape 150A des images de variance pour obtenir des images de variance corrigées sera ${σ}_{b , SVD}^{2} = 2 {N}_{trans} {σ}_{b , RF}^{2} - Δ {σ}_{SVD}^{2}$

Selon une deuxième méthode d'estimation du bruit, si le bruit ne suit pas cette loi, il est possible d'effectuer une correction alternative, par exemple en minimisant la norme L2 de l'image ${σ}_{j}^{2}$ reconstruite soumise à la soustraction d'une constante : ${σ}_{b} = arg {min}_{Q} {\sum }_{j = 1}^{{M}_{λ}} \left‖{σ}_{j}^{2}\left(r\right)-{Q}^{2}\right‖$

La valeur de la constante ${Q}^{2}$ minimisant cette norme permet de calculer la valeur ${σ}_{b , SVD}^{2}$ à soustraire à ${σ}_{j}^{2} \left(r\right)$

D'autres méthodes d'estimation du niveau de bruit sont utilisables.

Le procédé de traitement d'images photoacoustiques décrit dans ce document a été appliqué à des images volumiques acquises dans l'embryon de poulet et nous retrouvons des valeurs proches de taux d'oxygénation avec la spectroscopie photoacoustique conventionnelle basée sur une image moyenne dans les structures visibles.

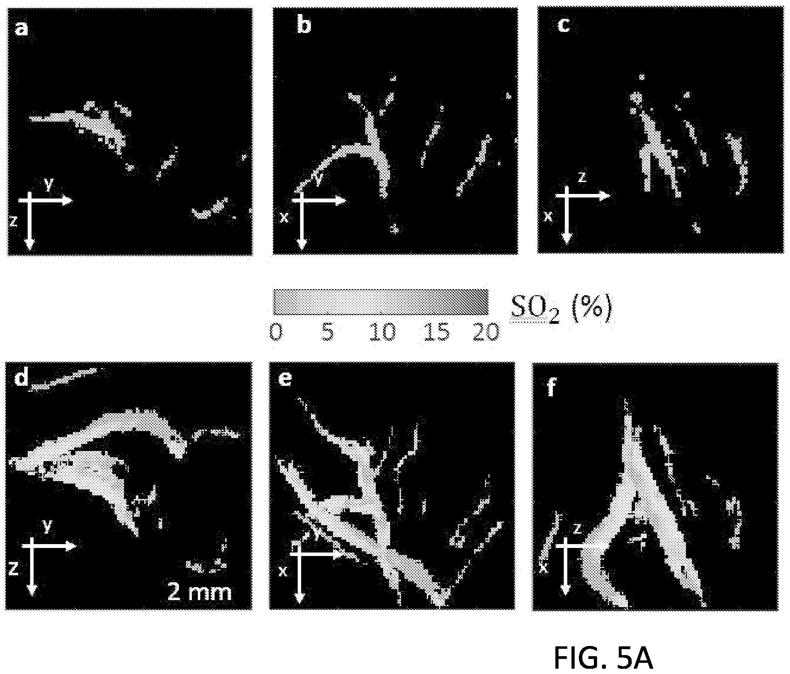

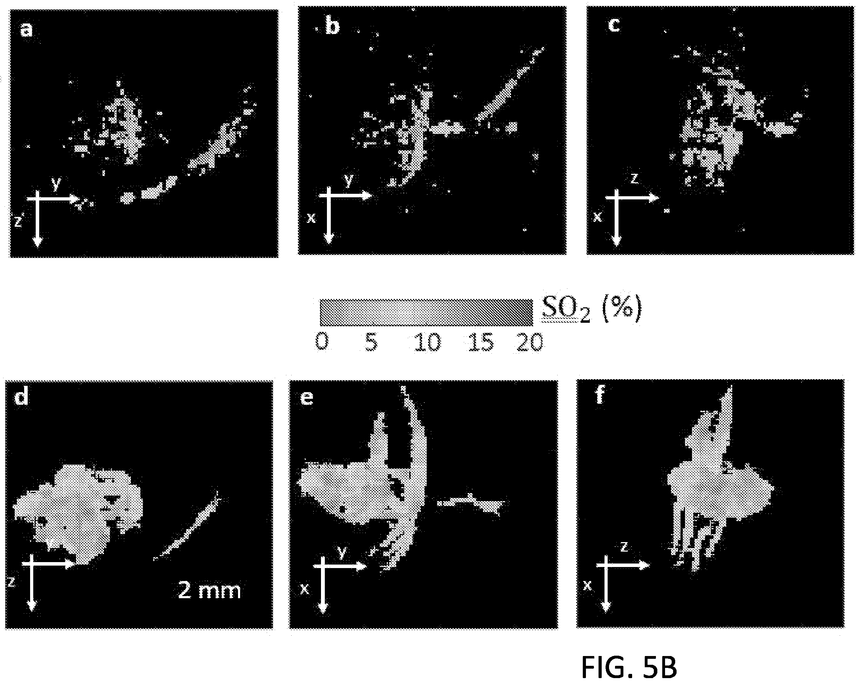

Les Figs. 5A-B montrent l'amélioration de la visibilité de certains éléments du tissu biologique par le procédé décrit dans ce document dans le cas de son application à l'imagerie de l'oxygénation des vaisseaux sanguins. Deux exemples sont illustrés. Dans les deux exemples (fig. 5A et fig. 5B), la première ligne correspond de gauche à droite, à une image d'oxygénation obtenue à partir de l'image photoacoustique moyenne m_{λ} selon trois plans de projection YZ (a), XY (b) et XZ (c). Ces images d'oxygénation ont une visibilité limitée. L'image 3D d'oxygénation obtenue à partir de l'imagerie des fluctuations d'absorption est montrée sur la deuxième ligne, avec une projection selon les mêmes plans YZ (d), XY (e) et XZ (f) où l'on voit beaucoup plus de structures qu'avec une technique d'imagerie photoacoustique conventionnelle.

Sur la fig. 5A, la zone imagée correspond à la membrane chorioallantoique. La technique d'imagerie des fluctuations d'absorption (fig. 5A, images d,e,f) permet d'étendre la mesure d'oxygénation à beaucoup plus de vaisseaux puisque certains n'apparaissaient pas sur l'image conventionnelle (fig. 5A, images a,b,c) du fait de leur orientation ou de leur taille.

Sur la fig. 5B, la zone imagée correspond au cœur de l'embryon de poulet. La technique par fluctuation (fig. 5B, images d,e,f) permet d'étendre la mesure d'oxygénation à l'ensemble de l'organe ainsi qu'aux vaisseaux sortants alors que très peu d'information apparait sur l'image conventionnelle (fig. 5B, images a,b,c) sous forme d'une distribution de pixels discontinue.

Dans le cas où le milieu est transparent (comme c'est le cas de l'embryon de poulet avec vascularisation entouré d'un milieu clair), il n'est pas nécessaire de corriger la coloration du spectre due à l'utilisation de plusieurs longueurs d'onde par le tissu biologique.

La méthode d'imagerie d'oxygénation des écoulements sanguins qui a été décrite ici repousse les limitations de détecteurs conventionnels avec des modifications mineures dans le matériel : il suffit d'acquérir suffisamment d'images (typiquement au moins 50) pour extraire la fluctuation d'intérêt et d'effectuer un post-traitement selon ce qui a été décrit ici.

Les fonctions, moteurs, schémas de principe, diagrammes de flux, diagrammes de transition d'état et/ou organigrammes présentés dans ce document représentent des vues conceptuelles de circuits illustratifs mettant en œuvre les principes de l'invention. De même, tous les organigrammes, diagrammes de flux, diagrammes de transition d'état, pseudo-codes et autres représentent divers aspects de procédé peuvent être essentiellement mis en œuvre par des instructions de programme informatique stockées sur un support lisible par ordinateur et ainsi être mis en œuvre par un processeur ou un dispositif incluant un processeur, que ce processeur ou dispositif soit ou non explicitement représenté.

Selon un mode de réalisation, une ou plusieurs ou toutes les étapes d'un procédé de traitement d'images photoacoustiques sont mises en œuvre par un logiciel ou programme informatique.

La présente description concerne ainsi un programme informatique susceptible d'être exécuté par un processeur de données, ce programme informatique comportant des instructions de programme informatique pour commander l'exécution par un dispositif d'une ou plusieurs ou de toutes les étapes d'un procédé de traitement d'images photoacoustiques selon l'un quelconque des modes de réalisation décrit dans ce document. Ces instructions de programme informatique sont destinées par exemple à être stockées dans une mémoire d'un dispositif, chargées puis exécutées par un processeur de ce dispositif.

Ce programme informatique peut utiliser n'importe quel langage de programmation, et être sous la forme de code source, code objet, ou de code intermédiaire entre code source et code objet, tel que dans une forme partiellement compilée, ou dans n'importe quelle autre forme souhaitable.

Le dispositif peut être mis en œuvre par une ou plusieurs machines physiquement distinctes et présente globalement l'architecture d'un ordinateur, incluant des constituants d'une telle architecture : mémoire(s) de données, processeur(s), bus de communication, interface(s) matérielle(s) pour la connexion de ce dispositif informatique à un réseau ou un autre équipement, interface(s) utilisateur, etc.

La présente description concerne aussi un support d'informations lisible par un processeur de données, et comportant des instructions d'un programme informatique tel que mentionné ci-dessus. Le support d'informations peut être n'importe quelle entité ou dispositif capable de stocker le programme.

Le support d'informations peut être n'importe quel moyen matériel, entité ou dispositif, capable de stocker un signal. Par exemple, le support peut comporter un moyen de stockage, tel qu'une mémoire ROM ou RAM, par exemple un disque CD ROM ou encore un moyen d'enregistrement magnétique, un disque dur d'ordinateur, des supports de stockage optiques, des dispositifs de mémoire flash et/ou d'autres supports tangibles lisibles par machine pour stocker des informations. Le terme "support lisible par ordinateur" peut inclure, sans s'y limiter, des dispositifs de stockage portables ou fixes, des dispositifs de stockage optiques et divers autres supports capables de stocker, de contenir ou de transporter des instructions et/ou des données.

Il peut s'agir de support de stockage informatique et/ou de supports de communication, ou plus généralement tout support qui facilite le transfert d'un programme informatique d'un endroit à un autre.

Des exemples de support lisible par ordinateur comprennent, sans s'y limiter, un lecteur flash ou d'autres dispositifs de mémoire flash (par exemple, des clés de mémoire, des bâtons de mémoire, un lecteur de clé USB), un CD-ROM ou un autre stockage optique, un DVD, un stockage sur disque magnétique ou d'autres dispositifs de stockage magnétique, une mémoire à l'état solide, une puce de mémoire, une mémoire vive, une mémoire ROM, une mémoire EEPROM, des cartes à puce, un système de gestion de base de données relationnelle, un système de gestion des données de l'entreprise, un système de gestion des données de l'entreprise, etc.

Le support d'informations peut être un support transmissible sous forme d'onde porteuse tel qu'un signal électromagnétique (signal électrique, radio ou optique), qui peut être acheminé via un moyen de transmission approprié, filaire ou non filaire: câble électrique ou optique, liaison radio ou infrarouge, ou par d'autres moyens.

Le terme « processeur » peut par exemple désigner tout microprocesseur, microcontrôleur, contrôleur, circuit intégré ou unité centrale de traitement (CPU) comprenant une ou plusieurs unités de traitement ou un ou plusieurs noyaux de traitement basé sur du hardware. De plus, le terme "processeur" ne doit pas être interprétée comme se référant exclusivement à un matériel capable d'exécuter des instructions de programme informatique, mais peut par exemple désigner un processeur de signaux numériques (DSP), un processeur de réseau, un circuit intégré spécifique à une application (ASIC), un réseau de portes programmable par l'utilisateur (FPGA), ou autre circuit qu'il soit programmable ou non, spécifique ou non. Le terme « processeur » peut également correspondre à une combinaison de plusieurs des exemples de réalisation mentionnés ici.

La présente description concerne un dispositif de traitement d'images photoacoustiques comprenant, au moins une mémoire de données comprenant des instructions de code de programme, au moins un processeur de données, le processeur de données étant configurées pour, lors que les instructions de code de programme sont exécutées par le processeur de données, causer l'exécution par le dispositif de traitement d'images photoacoustiques d'une ou plusieurs ou de toutes les étapes d'un procédé de traitement d'image photoacoustiques selon l'un quelconque des modes de réalisation décrit dans ce document.

Dans un mode de réalisation, le dispositif de traitement d'images photoacoustiques comprend:
- des moyens de stockage de données, par exemple une ou plusieurs mémoires, pour le stockage d'instructions de programme informatique conçues pour commander l'exécution d'une ou plusieurs ou de toutes les étapes d'un procédé de traitement d'image photoacoustiques selon l'un quelconque des modes de réalisation décrit dans ce document;
- des moyens de traitement de données, notamment un processeur de données, configuré pour exécuter les instructions de programme informatique afin de mettre en œuvre une ou plusieurs ou toutes les étapes d'un procédé de traitement d'image photoacoustiques selon l'un quelconque des modes de réalisation décrit dans ce document.

Plus généralement le dispositif de traitement d'images photoacoustiques comprend des moyens de mise en œuvre d'une ou plusieurs ou de toutes les étapes d'un procédé de traitement d'images photoacoustiques selon l'un quelconque des modes de réalisation décrit dans ce document. Ces moyens comprennent par exemple des moyens logiciels (software) et/ou matériels (hardware).

## Revendications

1. Procédé de traitement d'images photoacoustiques, comprenant
- une obtention (110) d'une succession temporelle d'images d'un échantillon acquises par un système d'imagerie photoacoustique pour M_{λ} longueurs d'ondes d'impulsions d'excitations avec N images acquises par longueur d'onde;
- un filtrage (120) spatio-temporel multispectral par décomposition en valeurs singulières appliqué à l'ensemble des N*M_{λ} images acquises de sorte à obtenir N*M_{λ} images filtrées ;
- pour chaque longueur d'onde, un calcul (140) d'une image de variance filtrée à partir des N images filtrées, un pixel de coordonnée r dans l'image de variance filtrée étant égal à la variance de la distribution des valeurs de pixels de même coordonnée r dans les images filtrées obtenues pour cette longueur d'onde ;
- pour chaque longueur d'onde, une correction (150A) de l'image de variance filtrée par soustraction d'une variance du bruit électronique résiduel après le filtrage spatio-temporel multispectral, produit par les capteurs d'ondes ultrasonores du système d'imagerie photoacoustique.

2. Procédé selon la revendication 1 dans lequel le procédé comprend pour chaque longueur d'onde, une détermination d'une image de fluctuations corrigée dont chaque pixel est égal à la racine carrée du pixel correspondant de l'image de variance corrigée obtenue par ladite soustraction pour la longueur d'onde considérée.

3. Procédé selon la revendication 1 ou 2, comprenant en outre une estimation de la variance du bruit électronique résiduel produit par les capteurs d'ondes ultrasonores sur les images, la variance du bruit électronique résiduel produit par les capteurs d'ondes ultrasonores sur les images étant estimée en fonction d'une variance du bruit électronique produit dans les signaux photoacoustiques acquis en l'absence d'échantillon corrigée par une quantité de bruit éliminée par le filtrage spatio-temporel multispectral par décomposition en valeurs singulières, la quantité de bruit éliminée étant estimée sur la base des valeurs singulières correspondant aux composantes de plus basse énergie supprimées par le filtrage spatio-temporel multispectral par décomposition en valeurs singulières.

4. Procédé selon l'une quelconque des revendications 1 à 3, comprenant pour chaque longueur d'onde considérée, une normalisation (150B) de l'image de fluctuations corrigée par une fonction de la fluence d'impulsions laser du système d'imagerie photoacoustique de sorte à obtenir une image de fluctuations d'absorption représentative des fluctuations d'absorption dues à l'échantillon.

5. Procédé selon l'une quelconque des revendications 1 à 4, dans lequel le filtrage (120) spatio-temporel multispectral par décomposition en valeurs singulières comprend une sélection des composantes correspondant aux valeurs singulières de plus haute énergie à supprimer et une suppression de composantes sélectionnées,
la sélection étant effectuée en choisissant parmi un ensemble de valeurs d'index un index identifiant la première composante à conserver pour lequel un rapport contraste à bruit est maximal,
le rapport contraste à bruit déterminé pour un index étant déterminé pour des images de variance filtrées calculées par filtrage (120) spatio-temporel multispectral par décomposition en valeurs singulières appliquant cet index pour identifier la première composante à conserver.

6. Procédé selon la revendication 5 dans lequel le rapport contraste à bruit est déterminé en éliminant le contraste dû à la valeur moyenne des images acquises pour au moins une longueur d'onde.

7. Procédé selon l'une quelconque des revendications 4 à 6 lorsqu'elle dépend de la revendication 4, le procédé comprenant un calcul (160) d'une image du taux de saturation en oxygène à partir d'au moins deux images de fluctuations d'absorption obtenues pour au moins deux longueurs d'onde correspondantes.

8. Procédé selon la revendication 7 dans lequel le calcul (160) d'une image du taux de saturation en oxygène est effectué sur la base d'un modèle exprimant pour chaque pixel de coordonnée r une relation entre une concentration totale en hémoglobine, un taux de saturation en oxygène et la valeur au pixel r de l'image de fluctuations d'absorption.

9. Dispositif de traitement d'images photoacoustiques comprenant, au moins une mémoire de données comprenant des instructions de code de programme, au moins un processeur de données, le processeur de données étant configurées pour, lors que les instructions de code de programme sont exécutées par le processeur de données, causer l'exécution par le dispositif de traitement d'images photoacoustiques d'un procédé selon l'une quelconque des revendications 1 à 8.

10. Support de données lisible par ordinateur comprenant des instructions de programme informatique qui, lorsqu'elles sont exécutées par un processeur, causent l'exécution d'un procédé selon l'une quelconque des revendications 1 à 8.

11. Programme informatique comprenant des instructions de programme informatique qui, lorsqu'elles sont exécutées par un processeur, causent l'exécution d'un procédé selon l'une quelconque des revendications 1 à 8.

## Patentansprüche

1. - Verarbeitungsverfahren photoakustischer Bilder, umfassend
- ein Erlangen (110) einer zeitlichen Abfolge von Bildern einer Probe, die von einem photoakustischen Bildgebungssystem für M_{λ} Erregungsimpulswellenlängen mit N pro Wellenlänge aufgenommenen Bildern erfasst werden;
- ein multispektrales raum-zeitliches Filtern (120) mittels Singulärwertzerlegung, angewendet auf die Gesamtheit der N* M_{λ} aufgenommenen Bilder, um N* M_{λ} gefilterte Bilder zu erlangen;
- für jede Wellenlänge, ein Berechnen (140) eines gefilterten Varianzbilds aus den N gefilterten Bildern, wobei ein Pixel mit Koordinate r im gefilterten Varianzbild gleich wie die Varianz der Verteilung der Pixelwerte mit derselben Koordinate r in den für diese Wellenlänge erlangten gefilterten Bildern ist;
- für jede Wellenlänge, ein Korrigieren (150A) des gefilterten Varianzbilds durch Subtraktion einer Varianz des nach dem multispektralen raum-zeitlichen Filtern verbleibenden elektronischen Rauschens, das von den Ultraschallwellensensoren des photoakustischen Bildgebungssystems erzeugt wird.

2. - Verfahren nach Anspruch 1, wobei das Verfahren für jede Wellenlänge ein Bestimmen eines korrigierten Fluktuationsbilds umfasst, dessen jedes Pixel gleich wie die Quadratwurzel des entsprechenden Pixels des durch die Subtraktion für die betrachtete Wellenlänge erlangten korrigierten Varianzbilds ist.

3. - Verfahren nach Anspruch 1 oder 2, ferner umfassend ein Schätzen der Varianz des von den Ultraschallwellensensoren auf den Bildern erzeugten verbleibenden elektronischen Rauschens, wobei die Varianz des von den Ultraschallwellensensoren auf den Bildern erzeugten verbleibenden elektronischen Rauschens abhängig von einer Varianz des in den photoakustischen Signalen in Abwesenheit einer Probe erzeugten elektronischen Rauschens, korrigiert um eine durch die multispektrale raum-zeitliche Filterung mittels Singulärwertzerlegung eliminierte Rauschmenge, geschätzt wird, wobei die eliminierte Rauschmenge basierend auf den Singulärwerten geschätzt wird, die den durch das multispektrale raum-zeitliche Filtern mittels Singulärwertzerlegung unterdrückten niederenergetischen Komponenten entsprechen.

4. - Verfahren nach einem der Ansprüche 1 bis 3, umfassend für jede betrachtete Wellenlänge eine Normalisierung (150B) des korrigierten Fluktuationsbilds durch eine Funktion der Laserimpuls-Fluenz des photoakustischen Bildgebungssystems, um ein Absorptionsfluktuationsbild zu erlangen, das die durch die Probe verursachten Absorptionsfluktuationen darstellt.

5. - Verfahren nach einem der Ansprüche 1 bis 4, wobei das multispektrale raum-zeitliche Filtern (120) mittels Singulärwertzerlegung eine Auswahl der zu unterdrückenden Komponenten mit den höchsten Singulärwerten und eine Unterdrückung ausgewählter Komponenten umfasst,
wobei die Auswahl erfolgt, indem aus einer Menge von Indexwerten ein Index ausgewählt wird, der die erste beizubehaltende Komponente identifiziert, für die das Kontrast-Rausch-Verhältnis maximal ist,
wobei das für einen Index bestimmte Kontrast-Rausch-Verhältnis für gefilterte Varianzbilder bestimmt wird, die durch multispektrales raum-zeitliches Filtern (120) mittels Singulärwertzerlegung unter Anwendung dieses Indexes zum Identifizieren der ersten beizubehaltenden Komponente berechnet werden.

6. - Verfahren nach Anspruch 5, wobei das Kontrast-Rausch-Verhältnis bestimmt wird, indem der Kontrast, der dem Mittelwert der für mindestens eine Wellenlänge aufgenommenen Bilder entspricht, eliminiert wird.

7. - Verfahren nach einem der Ansprüche 4 bis 6, wenn abhängig von Anspruch 4, das Verfahren umfassend ein Berechnen (160) eines Bilds des Sauerstoffsättigungsgrades anhand mindestens zweier Absorptionsfluktuationsbilder, die für mindestens zwei entsprechende Wellenlängen erlangt werden.

8. - Verfahren nach Anspruch 7, wobei das Berechnen (160) eines Bilds des Sauerstoffsättigungsgrades basierend auf einem Modell erfolgt, das für jedes Pixel mit Koordinate r eine Beziehung zwischen einer Gesamthämoglobinkonzentration, einem Sauerstoffsättigungsgrad und dem Wert an dem Pixel r des Absorptionsfluktuationsbilds ausdrückt.

9. - Verarbeitungsvorrichtung photoakustischer Bilder, umfassend mindestens einen Datenspeicher, umfassend Programmcodeanweisungen, mindestens einen Datenprozessor, wobei der Datenprozessor konfiguriert ist, um, wenn die Programmcodeanweisungen von dem Datenprozessor ausgeführt werden, die Ausführung eines Verfahrens nach einem der Ansprüche 1 bis 8 durch die Verarbeitungsvorrichtung photoakustischer Bilder zu bewirken.

10. - Computerlesbarer Datenträger, umfassend Computerprogrammanweisungen, die, wenn sie von einem Prozessor ausgeführt werden, die Ausführung eines Verfahrens nach einem der Ansprüche 1 bis 8 bewirken.

11. - Computerprogramm, umfassend Computerprogrammanweisungen, die, wenn sie von einem Prozessor ausgeführt werden, die Ausführung eines Verfahrens nach einem der Ansprüche 1 bis 8 bewirken.

## Claims

1. - A method for processing photoacoustic images, comprising
- obtaining (110) a temporal succession of images of a sample acquired by a photoacoustic imaging system for M_{λ} excitation pulse wavelengths with N images acquired per wavelength;
- multispectral spatio-temporal filtering (120) by singular value decomposition applied to all the N*M_{λ} images acquired so as to obtain N*M_{λ} filtered images;
- for each wavelength, calculating (140) a filtered variance image from the N filtered images, one pixel of coordinate r in the filtered variance image being equal to the variance of the distribution of pixel values of the same coordinate r in the filtered images obtained for that wavelength;
- for each wavelength, correcting (150A) the filtered variance image by subtracting a variance of the residual electronic noise after the multispectral spatio-temporal filtering produced by the ultrasonic wave sensors of the photoacoustic imaging system.

2. - The method according to claim 1, wherein the method comprises, for each wavelength, a determination of a corrected image of fluctuations, each pixel of which is equal to the square root of the corresponding pixel of the corrected variance image obtained by said subtraction for the wavelength considered.

3. - The method according to claim 1 or 2, further comprising estimating the variance of the residual electronic noise produced by the ultrasonic wave sensors on the images, the variance of the residual electronic noise produced by the ultrasonic wave sensors on the images being estimated as a function of a variance of the electronic noise produced in the photoacoustic signals acquired in the absence of a sample corrected by an amount of noise eliminated by the multispectral spatio-temporal filtering by decomposition into singular values, the amount of noise eliminated being estimated based on the singular values corresponding to the lowest energy components removed by the multispectral spatio-temporal filtering by singular value decomposition.

4. - The method according to any of claims 1 to 3, comprising for each wavelength considered, normalizing (150B) the image of fluctuations corrected by a function of the laser pulse fluence of the photoacoustic imaging system, so as to obtain an image of absorption fluctuations representative of the absorption fluctuations due to the sample.

5. - The method according to any of claims 1 to 4, wherein the multispectral spatio-temporal filtering (120) by singular value decomposition comprises selecting the components corresponding to the highest energy singular values to be removed and removing selected components,
the selection being made by choosing from a set of index values an index identifying the first component to be kept for which a contrast-to-noise ratio is maximum,
the contrast-to-noise ratio determined for an index being determined for filtered variance images calculated by multispectral spatio-temporal filtering (120) by decomposition into singular values applying the index in order to identify the first component to be kept.

6. - The method according to claim 5, wherein the contrast-to-noise ratio is determined by eliminating the contrast due to the mean value of the images acquired for at least one wavelength.

7. - The method according to any of claims 4 to 6 when dependent on claim 4, the method comprising calculating (160) an image of the oxygen saturation rate from at least two images of absorption fluctuations obtained for at least two corresponding wavelengths.

8. - The method according to claim 7, wherein the calculation (160) of an image of the oxygen saturation rate is performed based on a model expressing, for each pixel of coordinate r, a relation between a total hemoglobin concentration, an oxygen saturation rate and the value at pixel r of the image of absorption fluctuations.

9. - A photoacoustic image processing device comprising at least one data memory comprising program code instructions, at least one data processor, the data processor being configured, when the program code instructions are executed by the data processor, to make the photoacoustic image processing device execute a method according to any of claims 1 to 8.

10. - A computer-readable data storage medium including computer program instructions which, when executed by a processor, cause the execution of a method according to any of the claims 1 to 8.

11. - A computer program comprising computer program instructions which, when executed by a processor, cause the execution of a method according to any of the claims 1 to 8.
